# EUROPEAN PATENT APPLICATION

(11) **EP 4 002 201 A1**
(43) Date of publication of application: **25.05.2022**
(21) Application number: 21208975.9
(22) Date of filing: 18.11.2021
(51) Int. Cl.: G06K 9/00, G06T 7/00, A61B 5/00, G16H 30/40

(54) **BIOLOGICAL CLASSIFICATION DEVICE AND METHOD FOR ALZHEIMER'S DISEASE USING MULTIMODAL BRAIN IMAGE**

(30) Priority: 19.11.2020 KR 20200155063
(71) Applicant: HEURON CO., LTD., Incheon 21558 (KR); Gil Medical Center, Incheon 21565 (KR); Gachon University of Industry-Academic Cooperation Foundation, Seongnam-si, Gyeonggi-do 13120 (KR)
(72) Inventor: PARK, Seongbeom, 21659 Incheon (KR); SONG, Soohwa, 21584 Incheon (KR); NOH, Young, 06601 Seoul (KR)
(74) Representative: Prüfer & Partner mbB Patentanwälte · Rechtsanwälte

(57) **Abstract**

The present disclosure relates to a biological classification device and a method for Alzheimer's disease using a brain image. According to an aspect of the present disclosure, a biological classification device for Alzheimer's disease using a brain image includes an image receiving unit which receives a plurality of images obtained by capturing a brain of a subject; an image processing unit which acquires neurodegeneration feature related to the brain of the subject and SUVR information from the plurality of images; an image analysis unit which performs first determination of whether it is normal or abnormal with respect to cranial nerves based on the neurodegeneration feature and second determination and third determination of whether it is normal or abnormal with respect to beta amyloid protein and tau protein based on the SUVR information; and a classifying unit which performs biological classification of the subject related to the Alzheimer's disease using the first determination, the second determination, and the third determination together.

## Description

### CROSS-REFERENCE TO RELATED APPLICATIONS

This application claims the priority of Korean Patent Application No. 10-2020-0155063 filed on 19 November 2020, in the Korean Intellectual Property Office, the disclosure of which is incorporated herein by reference.

### BACKGROUND

### Field

The present disclosure relates to biological classification device and method for Alzheimer's disease using a brain image, and more particularly, to a device and a method of performing biological classification of Alzheimer's disease by analyzing an MRI image, an amyloid PET image, and a tau PET image related to the brain.

### Description of the Related Art

Alzheimer's disease which accounts for 50 to 60% of cases of dementia is the most widely known neurodegenerative disease. According to a recent report, approximately 50 million people suffer from dementia worldwide and are expected to increase to approximately 152 million by 2050.

Alzheimer's disease begins 20 years ago but changes in the brain may not be easily noticed until symptoms appear. The noticeable symptoms such as memory loss or speech impairment appear on the outside only after some changes in the brain have occurred. These symptoms are caused by the damage or destruction of nerve cells in the brain which involve in thinking, learning, and memory (cognitive function). As the disease progresses, other neurons in the brain are also damaged and destroyed, which eventually affects basic physical activities such as walking and swallowing.

Accordingly, it is very important to accurately diagnose Alzheimer's disease.

As a typical biological change of Alzheimer's disease, beta-amyloid (Aβ) which is a protein fragment outside the neurons and tau proteins which are abnormal proteins in the neurons are accumulated. This change disrupts the communication between neurons in the synapse to affect the damage and the death of the neurons.

In the past, Alzheimer's disease was diagnosed by performing various tests such as medical examination, neuropsychological test, and blood test and then collecting the test results and was confirmed only by post-mortem autopsy. However, in many cases, Alzheimer's disease may not be accurately diagnosed with only these tests.

Recently, in accordance with the development of technologies, PET which may test amyloid and hyperphosphorylated tau in the brain has been developed and deposition of beta-amyloid and abnormally phosphorylated tau neurofibrillary tangles which are typical features of Alzheimer's disease may be observed from living people.

Further, brain atrophy may be observed through MRI.

Currently, Alzheimer's disease is diagnosed when Alzheimer's disease related biomarkers which have been mentioned above are positive, regardless of a decline in the cognitive function.

That is, the certainty of unbiased diagnosis of Alzheimer's disease may be increased using amyloid PET, tau PET, and MRI.

### [Related Art Document]

### [Patent Document]

1. Korean Registered Patent No. 10-2020157 (published on November 4, 2019)
2. Korean Registered Patent No. 10-1995383 (published on July 2, 2019)

### SUMMARY

An object of the present disclosure is to provide a device and a method for performing biological classification related to Alzheimer's disease by determining and combining whether it is normal or abnormal with respect to a predetermined biomarker after acquiring a neurodegeneration feature related to the brain of a patient from an MRI image and acquiring standardized uptake value ratio (SUVR) images from an amyloid PET image and a tau PET image.

Specifically, an object of the present disclosure is to provide a device and a method for performing first determination of whether it is normal or abnormal with respect to a neurodegeneration feature biomarker, second determination of whether it is normal or abnormal with respect to an amyloid PET image biomarker, and determination of whether it is normal or abnormal with respect to a tau PET image biomarker, and performing biological classification related to Alzheimer's disease based on a combination of three determination results.

An object of the present disclosure is to provide a biological classification device and method including first classification indicating that a patient is a normal stage, second classification indicating that the patient corresponds to an early stage of Alzheimer's disease, third classification indicating that the patient corresponds to Alzheimer's disease, fourth classification indicating that the patient has another pathology as well as Alzheimer's disease, and fifth classification indicating that the patient has a pathology other than Alzheimer's disease to a user.

Further, an object of the present disclosure is to provide a device and a method for classifying an entire region of the brain into a plurality of regions based on an MRI image, acquiring a neurodegeneration feature from the plurality of classified brain regions and acquiring an SUVR image from an amyloid PET image and an SUVR image from a tau PET image based on the plurality of classified brain regions to a user.

Further, an object of the present disclosure is to provide a device and a method for classifying and analyzing a brain of a patient into a plurality of regions based on an MRI image by applying a deep neural network module trained using at least one of a first model trained with a brain image in an axial direction and labelling data, a second model trained with a brain image in a coronal direction and the labelling data, and a third model trained with a brain image in a sagittal direction and the labelling data to a user.

Further, an object of the present disclosure is to provide a device and a method for acquiring an SUVR image from an amyloid PET image and a tau PET image based on region of interest (ROI) information acquired from an operation of a device of processing an MRI image in a plurality of classified brain regions to a user.

Further, an object of the present disclosure is to provide a device and a method for performing pre-processing such as partial volume correction (PVC) processing and co-registration processing, with regard to an amyloid PET image and a tau PET image to a user.

Further, an object of the present disclosure is to provide a device, a system, and a method which increase a probability of successful clinical trials by utilizing biological classification of Alzheimer's disease using a brain image to screen a patient group and a normal group.

In the meantime, technical objects to be achieved in the present invention are not limited to the aforementioned technical objects, and another not-mentioned technical object will be obviously understood by those skilled in the art from the description below.

In order to achieve the above-described technical objects, according to an aspect of the present disclosure, a biological classification device for Alzheimer's disease using a brain image may include an image receiving unit which receives a plurality of images obtained by capturing a brain of a subject; an image processing unit which acquires neurodegeneration feature related to the brain of the subject and SUVR information from the plurality of images; an image analysis unit which performs first determination of whether it is normal or abnormal with respect to cranial nerves based on the neurodegeneration feature and second determination and third determination of whether it is normal or abnormal with respect to beta amyloid protein and tau protein based on the SUVR information; and a classifying unit which performs biological classification of the subject related to the Alzheimer's disease using the first determination, the second determination, and the third determination together.

Further, the plurality of images may include an MRI image related to a brain of the subject and an amyloid PET image and a tau PET image related to the brain of the subject.

Further, the SUVR information includes a first SUVR image related to the amyloid PET image and a second SUVR image related to the tau PET image, and the image processing unit may classify the entire region of the brain of the subject into a plurality of regions and acquire the neurodegeneration feature, the first SUVR image, and the second SUVR image from the plurality of classified brain regions.

Further, the image analysis unit may include a first image analysis unit which performs first determination of whether it is normal or abnormal with regard to the cranial nerves based on the acquired neurodegeneration feature; a second image analysis unit which performs second determination of whether it is normal or abnormal with regard to beta amyloid protein based on the first SUVR image; and a third image analysis unit which performs third determination of whether it is normal or abnormal with regard to tau protein based on the second SUVR image.

Further, the biological classification performed by the classifying unit may include first classification indicating that a subject is a normal stage, second classification indicating that the subject is in an early stage of Alzheimer's disease, third classification indicating that the subject corresponds to Alzheimer's disease, fourth classification indicating that the subject has another pathology as well as Alzheimer's disease, and fifth classification indicating that the subject has a pathology other than Alzheimer's disease.

Further, the classifying unit may perform first classification of the subject when the first determination is normal, the second determination is normal, and the third determination is normal, second classification of the subject when the first determination is normal, the second determination is abnormal, and the third determination is normal, third classification of the subject when the first determination is normal, the second determination is abnormal, and the third determination is abnormal and when the first determination is abnormal, the second determination is abnormal, and the third determination is abnormal, fourth classification of the subject when the first determination is abnormal, the second determination is abnormal, and the third determination is normal, and fifth classification of the subject when the first determination is normal, the second determination is normal, and the third determination is abnormal, when the first determination is abnormal, the second determination is normal, and the third determination is normal, and when the first determination is abnormal, the second determination is normal, and the third determination is abnormal.

Further, the image processing unit may include: a first image processing unit which classifies the entire region of the brain of the subject into a plurality of regions based on the MRI image related to the brain of the subject and acquires the neurodegeneration feature from the plurality of classified brain regions; a second image processing unit which acquires the first SUVR image from the amyloid PET image related to the brain of the subject, based on the plurality of classified brain regions; and a third image processing unit which acquires the second SUVR image from the tau PET image related to the brain of the subject, based on the plurality of classified brain regions.

Further, the first image processing unit may include: a deep neural network module which is trained using at least one of a first model trained with a brain image in an axial direction and labelling data, a second model trained with a brain image in a coronal direction and the labelling data, and a third model trained with a brain image in a sagittal direction and the labelling data; a classification module which classifies the entire region of the brain of the subject into a plurality of regions based on the MRI image; and an analysis module which acquires neurodegeneration feature related to the brain of the subject based on the plurality of classified brain region.

Further, the analysis module may generate a neurodegeneration feature map based on the classified brain regions and acquires the neurodegeneration information from the neurodegeneration feature map and the neurodegeneration feature may include a cortical thickness, a volume, a surface area, and a gyrification index.

Further, the classification module may classify the entire region of the brain of the subject into a plurality of regions using any one of the first MRI image classified with respect to the axial direction by the first model, the second MRI image classified with respect to the coronal direction by the second model, and the third MRI image classified with respect to the sagittal direction by the third model.

Further, the deep neural network module may three-dimensionally reconstruct the MRI image using all the first MRI image classified with respect to the axial direction by the first model, the second MRI image classified with respect to the coronal direction by the second model, and the third MRI image classified with respect to the sagittal direction by the third model.

Further, the classification module may classify the entire region of the brain of the subject into 95 classes based on the MRI image which is three-dimensionally reconstructed and classify a hippocampus region among the 95 classes into 13 sub regions again.

Further, the classification module may reclassify the region which is classified into 95 classes and the hippocampus region which is classified into 13 sub regions again into a composite region by at least one of a task of additionally classifying into two or more regions and a task of composing two or more of the classified regions.

Further, the classification module may select and reclassify only regions related to a predetermined brain disease excluding regions which are not related to the predetermined brain disease from the region which is classified into 95 classes and the hippocampus region which is classified into 13 sub regions again.

Further, the analysis module may calculate a regional volume from the region, which is classified into 95 classes, a subfield volume from the hippocampus region which is classified into 13 sub regions again, and a composite region volume from the composite region.

Further, the second image processing unit and the third image processing unit may additionally apply region of interest (ROI) information used for the region classifying operation and the neurodegeneration feature operation of the first image processing unit to acquire the first SUVR image and the second SUVR image.

Further, the ROI may include a cerebellum grey matter region, a cerebellum white matter region, a whole cerebellum region, a pons region, and a brainstem region and the ROI used in the second image processing unit and the third image processing unit may vary depending on a tracer of the amyloid PET image and the tau PET image.

Further, the second image processing unit and the third image processing unit may perform a predetermined pre-processing process, and the pre-processing process may include partial volume correction (PVC) processing and co-registration processing.

The partial volume correction (PVC) processing is performed to correct a spill-out phenomenon that an image is blurred due to a resolution lower than a predetermined reference by the influence of a partial volume effect so that a concentration is measured to be low and a spill-in phenomenon that when the concentration around the region of interest is high, the concentration is measured to be higher than an actual concentration in the region of interest and the partial volume correction (PVC) processing method may include a geometric transfer matrix method and a Muller-Gartner method.

In order to achieve the above-described technical objects, according to another aspect of the present disclosure, a biological classification method for Alzheimer's disease using a brain image may include a first step of receiving a plurality of images obtained by capturing a brain of a subject, by an image receiving unit; a second step of acquiring a neurodegeneration feature related to the brain of the subject and SUVR information from the plurality of images, by an image processing unit; a third step of performing first determination of whether it is normal or abnormal with respect to cranial nerves based on the neurodegeneration feature and second determination and third determination of whether it is normal or abnormal with respect to beta amyloid protein and tau protein based on the SUVR information, by an image analysis unit; and a fourth step of performing biological classification of the subject related to the Alzheimer's disease using the first determination, the second determination, and the third determination together, by the classifying unit.

Further, the plurality of images may include an MRI image related to a brain of the subject and an amyloid PET image and a tau PET image related to the brain of the subject.

Further, the SUVR information includes a first SUVR image related to the amyloid PET image and a second SUVR image related to the tau PET image, and the image processing unit may classify the entire region of the brain of the subject into a plurality of regions and acquire the neurodegeneration feature, the first SUVR image, and the second SUVR image from the plurality of classified brain regions.

Further, the third step may include: a 3-1 step of performing first determination of whether it is normal or abnormal with regard to the cranial nerves based on the acquired neurodegeneration feature, by a first image analysis unit of the image analysis unit; a 3-2 step of performing second determination of whether it is normal or abnormal with regard to the beta amyloid protein based on the first SUVR image, by a second image analysis unit of the image analysis unit; and a 3-3 step of performing third determination of whether it is normal or abnormal with regard to the tau protein based on the second SUVR image, by a third image analysis unit of the image analysis unit.

In the fourth step, the biological classification performed by the classifying unit may include first classification indicating that a subject is a normal stage, second classification indicating that the subject is in an early stage of Alzheimer's disease, third classification indicating that the subject corresponds to Alzheimer's disease, fourth classification indicating that the subject has another pathology as well as Alzheimer's disease, and fifth classification indicating that the subject has a pathology other than Alzheimer's disease.

Further, in the fourth step, the classifying unit may perform first classification of the subject when the first determination is normal, the second determination is normal, and the third determination is normal, second classification of the subject when the first determination is normal, the second determination is abnormal, and the third determination is normal, third classification of the subject when the first determination is normal, the second determination is abnormal, and the third determination is abnormal and when the first determination is abnormal, the second determination is abnormal, and the third determination is abnormal, fourth classification of the subject when the first determination is abnormal, the second determination is abnormal, and the third determination is normal, and fifth classification of the subject when the first determination is normal, the second determination is normal, and the third determination is abnormal, when the first determination is abnormal, the second determination is normal, and the third determination is normal, and when the first determination is abnormal, the second determination is normal, and the third determination is abnormal.

Further, the second step may include a 2-1 step of classifying the entire region of the brain of the subject into a plurality of regions based on the MRI image related to the brain of the subject and acquiring the neurodegeneration feature from the plurality of classified brain regions, by a first image processing unit of the image processing unit; a 2-2 step of acquiring the first SUVR image from the amyloid PET image related to the brain of the subject, based on the plurality of classified brain regions, by a second image processing unit of the image processing unit; and a 2-3 step of acquiring the second SUVR image from the tau PET image related to the brain of the subject, based on the plurality of classified brain regions, by a third image processing unit of the image processing unit.

Further, the 2-1 step may include training a deep neural network module of the first image processing unit using at least one of a first model trained with a brain image in an axial direction and labelling data, a second model trained with a brain image in a coronal direction and the labelling data, and a third model trained with a brain image in a sagittal direction and the labelling data; classifying an entire region of a brain of the subject based on the MRI image into a plurality of regions, by a classification module of the first image processing unit; and acquiring a neurodegeneration feature related to the brain of the subject, based on the plurality of classified brain regions, by an analysis module of the first image processing unit.

Further, the analysis module may generate a neurodegeneration feature map based on the classified brain regions and acquires the neurodegeneration information from the neurodegeneration feature map and the neurodegeneration feature may include a cortical thickness, a volume, a surface area, and a gyrification index.

Further, the classification module may classify the entire region of the brain of the subject into a plurality of regions using any one of the first MRI image classified with respect to the axial direction by the first model, the second MRI image classified with respect to the coronal direction by the second model, and the third MRI image classified with respect to the sagittal direction by the third model.

Further, the deep neural network module may three-dimensionally reconstruct the MRI image using all the first MRI image classified with respect to the axial direction by the first model, the second MRI image classified with respect to the coronal direction by the second model, and the third MRI image classified with respect to the sagittal direction by the third model.

Further, the second image processing unit and the third image processing unit may additionally apply region of interest (ROI) information used for the region classifying operation and the neurodegeneration feature operation of the first image processing unit to acquire the first SUVR image and the second SUVR image.

Further, the ROI includes a cerebellum grey matter region, a cerebellum white matter region, a whole cerebellum region, a pons region, and a brainstem region, and the ROI used in the second image processing unit and the third image processing unit may vary depending on a tracer of the amyloid PET image and the tau PET image.

Further, the second image processing unit and the third image processing unit may perform a predetermined pre-processing process, and the pre-processing process may include partial volume correction (PVC) processing and co-registration processing.

In the meantime, according to still another aspect of the present disclosure, a method of increasing a probability of successful clinical trials by screening a subject group using a biological classification device for Alzheimer's disease using a brain image which includes an image receiving unit, an image processing unit, an image analysis unit, a classifying unit, and a central control unit may include a first step of receiving a plurality of images obtained by capturing brains of a plurality of subjects which is a candidate group of a clinical trial for proving a drug efficacy, by the image receiving unit; a second step of acquiring a neurodegeneration feature related to the brain of the plurality of subjects and SUVR information from the plurality of images, by the image processing unit; a third step of performing first determination of whether it is normal or abnormal with respect to cranial nerves based on the neurodegeneration feature and second determination and third determination of whether it is normal or abnormal with respect to beta amyloid protein and tau protein based on the SUVR information, by the image analysis unit; a fourth step of performing biological classification of the plurality of subjects related to the Alzheimer's disease using the first determination, the second determination, and the third determination together, by the classifying unit; a fifth step of providing the biological classification information of the plurality of subjects from the classifying unit to the central control unit; and a sixth step of screening a first subject for the clinical trial based on the biological classification information of the plurality of subjects, by the central control unit.

In the meantime, according to still another aspect of the present disclosure, a method of increasing a probability of successful clinical trials by screening a subject group using a biological classification device for Alzheimer's disease using a brain image which includes an image receiving unit, an image processing unit, an image analysis unit, a classifying unit, and a server which communicates with the biological classification device for Alzheimer's disease may include a first step of receiving a plurality of images obtained by capturing brains of a plurality of subjects which is a candidate group of a clinical trial for proving a drug efficacy, by the image receiving unit; a second step of acquiring a neurodegeneration feature related to the brain of the plurality of subjects and SUVR information from the plurality of images, by the image processing unit; a third step of performing first determination of whether it is normal or abnormal with respect to cranial nerves based on the neurodegeneration feature and second determination and third determination of whether it is normal or abnormal with respect to beta amyloid protein and tau protein based on the SUVR information, by the image analysis unit; a fourth step of performing biological classification of the plurality of subjects related to the Alzheimer's disease using the first determination, the second determination, and the third determination together, by the classifying unit; a fifth step of providing the biological classification information of the plurality of subjects from the classifying unit to the server; and a sixth step of screening a first subject for the clinical trial based on the biological classification information of the plurality of subjects, by the server.

As described above, according to the present disclosure, it is possible to provide a device and a method of performing biological classification related to Alzheimer's disease by determining and combining whether it is normal or abnormal with respect to a predetermined biomarker after acquiring a neurodegeneration feature related to the brain of a patient from an MRI image and acquiring a standardized uptake value ratio (SUVR) image from an amyloid PET image and a tau PET image.

Specifically, the present disclosure may provide a device and a method for performing first determination of whether it is normal or abnormal with respect to a neurodegeneration feature biomarker, second determination of whether it is normal or abnormal with respect to an amyloid PET image biomarker, and determination of whether it is normal or abnormal with respect to a tau PET image biomarker, and performing biological classification regard to Alzheimer's disease based on a combination of three determination results.

Further, the present disclosure may provide a biological classification device and method including first classification indicating that a patient is a normal stage, second classification indicating that the patient is in an early stage of Alzheimer's disease, third classification indicating that the patient corresponds to Alzheimer's disease, fourth classification indicating that the patient has another pathology as well as Alzheimer's disease, and fifth classification indicating that the patient has a pathology other than Alzheimer's disease to a user.

Further, the present disclosure may provide a device and a method for classifying an entire region of the brain into a plurality of regions based on an MRI image, acquiring a neurodegeneration feature from the plurality of classified brain regions and, at the same time, acquiring an SUVR image from an amyloid PET image and an SUVR image from a tau PET image based on the plurality of classified brain regions to a user.

Further, the present disclosure may provide a device and a method for classifying and analyzing a brain of a patient into a plurality of regions based on an MRI image by applying a deep neural network module trained using at least one of a first model trained with a brain image in an axial direction and labelling data, a second model trained with a brain image in a coronal direction and the labelling data, and a third model trained with a brain image in a sagittal direction and the labelling data to a user.

Further, the present disclosure may provide a device and a method for acquiring an SUVR image from an amyloid PET image and a tau PET image based on region of interest (ROI) information acquired from an operation of a device of processing an MRI image in a plurality of classified brain regions to a user.

Further, the present disclosure may provide a device and a method for performing pre-processing such as partial volume correction (PVC) processing and co-registration processing, with regard to an amyloid PET image and a tau PET image to a user.

Further, the present disclosure may provide a device, a system, and a method which increase a probability of successful clinical trials by utilizing biological classification of Alzheimer's disease using a brain image to screen a patient group and a normal group.

In the meantime, a technical object to be achieved in the present disclosure is not limited to the aforementioned effects, and other not-mentioned effects will be obviously understood by those skilled in the art from the description below.

### BRIEF DESCRIPTION OF THE DRAWINGS

The above and other aspects, features and other advantages of the present disclosure will be more clearly understood from the following detailed description taken in conjunction with the accompanying drawings, in which:
FIG. 1 illustrates an example of a block diagram of a biological classification device for Alzheimer's disease using a brain image according to the present disclosure;
FIG. 2 illustrates an example of a block diagram including a function of a biological classification device for Alzheimer's disease using a brain image according to the present disclosure;
FIG. 3 is a flowchart explaining for a biological classification method for Alzheimer's disease using a brain image according to the present disclosure;
FIG. 4 is a view explaining for a function of an image processing unit according to the present disclosure;
FIG. 5 is a view explaining for a function of an MRI image processing unit according to the present disclosure;
FIG. 6 illustrates an example of a block diagram of an MRI image processing unit according to the present disclosure
FIG. 7 is a view explaining for a function of a trained deep neural network module according to the present disclosure;
FIGS. 8A and 8B are views explaining for a process of classifying a brain region into a plurality of regions based on a trained deep neural network module and acquiring a neurodegeneration feature by an image processing unit, according to the present disclosure;
FIG. 9 is a view explaining for a process of acquiring SUVR images from an amyloid PET image and a tau PET image based on a plurality of classified brain regions, according to the present disclosure;
FIG. 10 is a view explaining for a process of acquiring an SUVR image based on region of interest (ROI) information in accordance with an operation of an MRI image processing unit in a plurality of classified brain regions, according to the present disclosure;
FIG. 11 is a table obtained by summarizing biological classification contents performed by a classifying unit as a table, according to the present disclosure;
FIG. 12 is a view illustrating a process of biologically classifying Alzheimer's disease by analyzing an MRI image, an amyloid PET image, and a tau PET image related to the brain, according to the present disclosure;
FIG. 13 is a view explaining for a method of increasing a probability of successful clinical trials by utilizing biological classification of Alzheimer's disease using a brain image to screen a patient group and a normal group, according to the present disclosure; and
FIG. 14 is a view explaining for another method of increasing a probability of successful clinical trials by utilizing biological classification of Alzheimer's disease using a brain image to screen a patient group and a normal group, according to the present disclosure.

### DETAILED DESCRIPTION OF THE PREFERRED EMBODIMENT

Hereinafter, an exemplary embodiment of the present disclosure will be described with reference to the accompanying drawings. The exemplary embodiments which will be described below do not unduly limit the contents of the present disclosure as set forth in the claims and the entire configuration described in the present embodiment may not be said to be essential as a solution for the present disclosure.

Hereinafter, a device and a method of performing biological classification of Alzheimer's disease using a brain image according to an exemplary embodiment of the present invention will be described in detail with reference to the accompanying drawings.

### Biological classification device for Alzheimer's disease using brain image

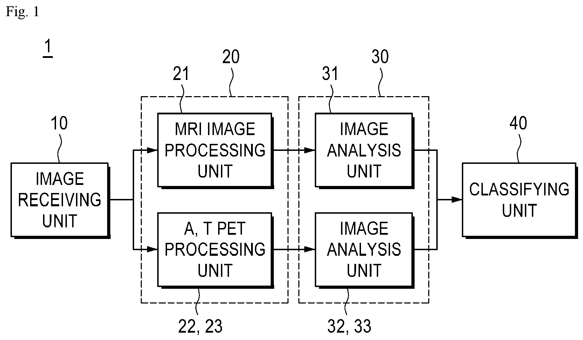

FIG. 1 illustrates an example of a block diagram of a biological classification device for Alzheimer's disease using a brain image according to the present disclosure.

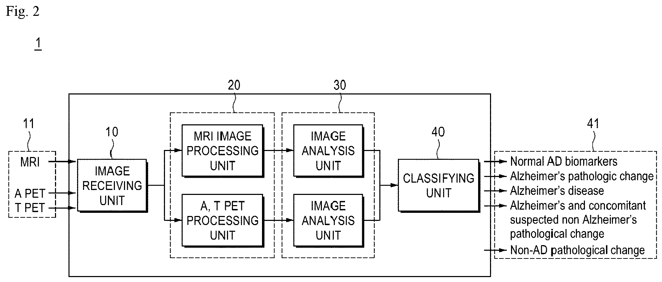

Further, FIG. 2 illustrates an example of a block diagram including a function of a biological classification device for Alzheimer's disease using a brain image according to the present disclosure.

Referring to FIG. 1, a biological classification device for Alzheimer's disease 1 using a brain image according to the present disclosure may include an image receiving unit 10, an image processing unit 20, an image analysis unit 30, and a classifying unit 40.

Here, the image receiving unit 10 receives a plurality of images obtained by capturing a brain of a patient.

Referring to FIG. 2, the plurality of images 11 received by the image receiving unit 10 may include an MRI image related to the brain of the patient and an amyloid PET image and a tau PET image related to the brain of the patient.

Returning to FIG. 1, the image processing unit 20 may acquire neurodegeneration feature related to a brain of a patient and a standardized uptake value ratio (SUVR) image from a plurality of images.

The image processing unit 20 according to the present disclosure may include an MRI image processing unit 21 which acquires neurodegeneration feature related to a brain of a patient from a plurality of images and A and T PET image processing units 22 and 23 which acquire standardized uptake value ratio (SUVR) images from an amyloid PET image and a tau PET image related to the brain of the patient.

Further, the image analysis unit 30 may determine whether it is normal or abnormal with respect to a plurality of predetermined biomarkers.

Specifically, the image analysis unit 30 according to the present disclosure may include an MRI image analysis unit 31, an amyloid PET image analysis unit 32, and a tau PET image analysis unit 33.

The MRI image analysis unit 31 makes first determination of whether it is normal or abnormal with respect to a predetermined neurodegeneration feature biomarker.

Further, the amyloid PET image analysis unit 32 makes second determination of whether it is normal or abnormal with respect to a predetermined amyloid PET image biomarker.

Further, the tau PET image analysis unit 33 makes third determination of whether it is normal or abnormal with respect to a predetermined tau PET image biomarker.

Next, the classifying unit 40 performs biological classification of a patient related to Alzheimer's disease using the first determination, the second determination, and the third determination of the image analysis unit 30.

Referring to FIG. 2, typically, the biological classification determined by the classifying unit 40 may include first classification indicating that a patient is in a normal stage, second classification indicating that the patient corresponds to an early stage of Alzheimer's disease, third classification indicating that the patient corresponds to Alzheimer's disease, fourth classification indicating that the patient has another pathology as well as Alzheimer's disease, and fifth classification indicating that the patient has a pathology other than Alzheimer's disease.

### Biological classification method for Alzheimer's disease using brain image

A biological classification method for Alzheimer's disease proposed by the present disclosure will be described based on a configuration of the biological classification device for Alzheimer's disease 1 using a brain image which has been described with respect to FIGS. 1 and 2.

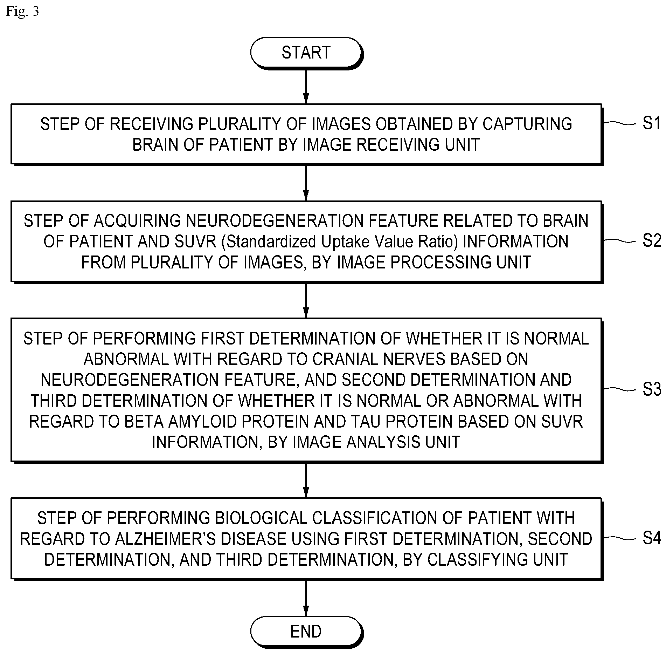

FIG. 3 is a flowchart explaining for a biological classification method for Alzheimer's disease using a brain image according to the present disclosure.

Referring to FIG. 3, a step S1 of receiving a plurality of images obtained by capturing a brain of a patient by the image receiving unit 10 is performed.

In the step S1, the plurality of images 11 received by the image receiving unit 10 may include an MRI image related to the brain of the patient, an amyloid PET image and a tau PET image related to the brain of the patient.

Next, a step S2 of acquiring a neurodegeneration feature with regard to the brain of the patient and the standardized uptake value ratio (SUVR) image from the plurality of images by the image processing unit 20 is performed.

In the step S2, the MRI image processing unit 21 acquires the neurodegeneration feature related to the brain of the patient from the plurality of images and the A and T PET image processing units 22 and 23 may acquire SUVR images from the amyloid PET image and the tau PET image related to the brain of the patient, respectively.

Next, a step S3 of performing first determination of whether it is normal or abnormal with respect to a predetermined neurodegeneration feature biomarker, second determination of whether it is normal or abnormal with respect to a predetermined amyloid PET image biomarker, and third determination of whether it is normal or abnormal with respect to a predetermined tau PET image biomarker by the image analysis unit 30 is performed.

In the step S3, the MRI image analysis unit 31 makes first determination of whether it is normal or abnormal with respect to a predetermined neurodegeneration feature biomarker, second determination of whether it is normal or abnormal with respect to a predetermined amyloid PET image biomarker, and third determination of whether it is normal or abnormal with respect to a predetermined tau PET image biomarker.

After the step S3, a step S4 of performing biological classification of the patient related to Alzheimer's disease using the first determination, the second determination, and the third determination by the classifying unit 40 is performed.

In the step S4, the biological classification determined by the classifying unit 40 includes first classification indicating that a patient is in a normal stage, second classification indicating that the patient corresponds to an early stage of Alzheimer's disease, third classification indicating that the patient corresponds to Alzheimer's disease, fourth classification indicating that the patient has another pathology as well as Alzheimer's disease, and fifth classification indicating that the patient has a pathology other than Alzheimer's disease.

Hereinafter, the image receiving unit 10, the image processing unit 20, the image analysis unit 30, and the classifying unit 40 which have been described based on FIGS. 1 and 2 and the steps of the biological classification method for Alzheimer's disease which has been described based on FIG. 3 will be described in more detail with reference to the drawings.

### Image processing unit

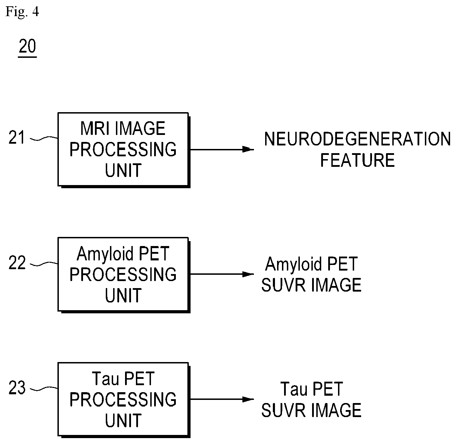

FIG. 4 is a view explaining for a function of an image processing unit according to the present disclosure.

Referring to FIG. 4, the image processing unit 20 according to the present disclosure may acquire neurodegeneration feature related to a brain of a patient and a standardized uptake value ratio (SUVR) image from a plurality of images.

Referring to FIG. 4, the image processing unit 20 according to the present disclosure may include an MRI image processing unit 21, an amyloid PET image processing unit 22, and a tau PET image processing unit 23.

First, the MRI image processing unit 21 may acquire neurodegeneration feature related to the brain of the patient from a plurality of images.

Next, the amyloid PET image processing unit 22 may acquire a first SUVR image from the amyloid PET image related to the brain of the patient.

Further, the tau PET image processing unit 23 may acquire a second SUVR image from the tau PET image related to the brain of the patient.

In this case, the MRI image processing unit 21 classifies the entire region of the brain into a plurality of regions based on the MRI image related to the brain of the patient and the amyloid PET image processing unit 22 and the tau PET image processing unit 23 acquire the first SUVR image and the second SUVR image, as well as the neurodegeneration feature, from the plurality of classified brain regions.

### MRI image processing unit

With regard to FIG. 4, first, the MRI image processing unit 21 will be described in detail.

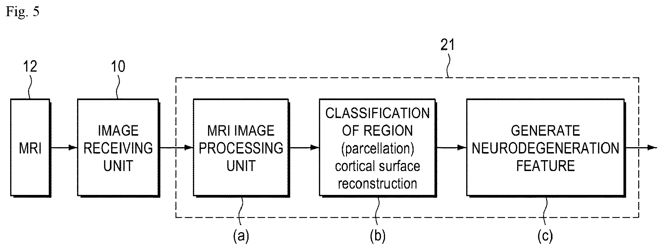

FIG. 5 is a view explaining for a function of an MRI image processing unit according to the present disclosure.

Referring to FIG. 5, the image receiving unit 10 receives the MRI image, and the MRI image processing unit 21 classifies the entire region of the brain into a plurality of regions (b), based on the MRI image related to the brain of the patient transmitted from the image receiving unit 10 (a), and generates the neurodegeneration feature from the plurality of classified brain regions (c).

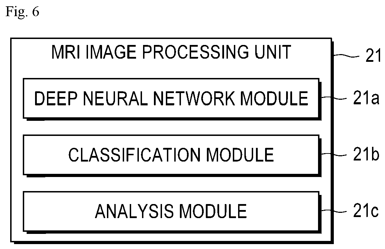

Specifically, FIG. 6 illustrates an example of a block diagram of an MRI image processing unit according to the present disclosure.

Referring to FIG. 6, the MRI image processing unit 21 according to the present disclosure may include a deep neural network module 21a, a classification module 21b, and an analysis module 21c.

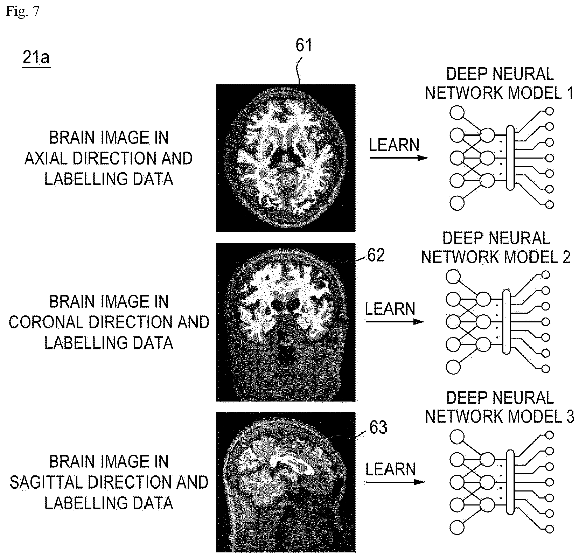

FIG. 7 is a view explaining for a function of a trained deep neural network module according to the present disclosure.

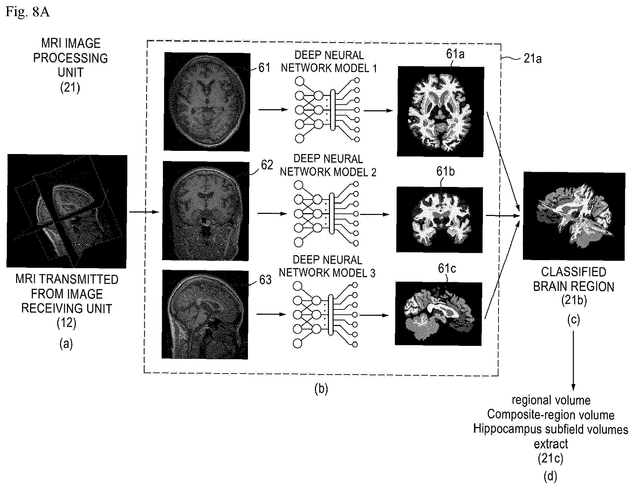

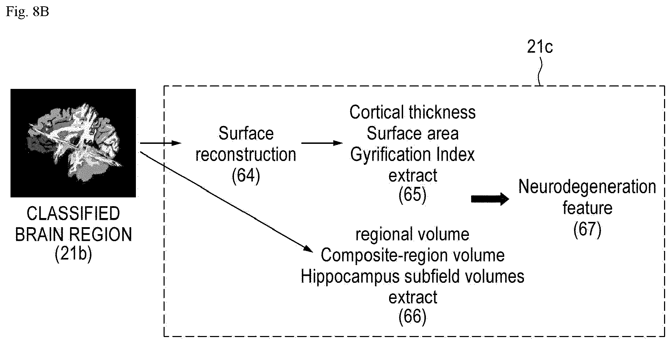

Further, FIGS. 8A and 8B are views explaining for a process of classifying a brain region into a plurality of regions based on a trained deep neural network module by an image processing unit and acquiring a neurodegeneration feature, according to the present disclosure.

Referring to FIG. 7, first, the deep neural network module 21a is trained using at least one of a first model trained with a brain image in an axial direction and labelling data 61, a second model trained with a brain image in a coronal direction and the labelling data 62, and a third model trained with a brain image in a sagittal direction and the labelling data 63.

Specifically, referring to FIG. 8A, the deep neural network module 21a may classify the entire region of the brain of a subject into a plurality of regions 21b using any one of a first MRI image 61a classified with respect to the axial direction by the first model, a second MRI image 61b classified with respect to the coronal direction by the second model, and a third MRI image 61c classified with respect to the sagittal direction by the third model.

Further, an image which is three-dimensionally reconstructed may be used by using the results of all the first model, the second model, and the third model.

That is, according to the present disclosure, the deep neural network module 21a may three-dimensionally reconstruct the MRI image using all a first MRI image classified with respect to the axial direction by the first model, a second MRI image classified with respect to the coronal direction by the second model, and a third MRI image classified with respect to the sagittal direction by the third model.

Further, the classification of the entire region of the brain of the patient into a plurality of regions based on the MRI image transmitted from the deep neural network module 21a by the classification module 21 will be described in more detail.

The classification module 21b may classify the entire region of the brain of the patient into 95 classes based on the three-dimensionally reconstructed MRI image by means of the deep neural network module 21a.

Further, the classification module 21b may reclassify a hippocampus region among 95 classes into 13 sub regions again.

Moreover, the classification module 21b may reclassify the region which is classified into 95 classes and the hippocampus region which is classified into 13 sub regions again into a composite region by at least one of a task of additionally classifying the regions into two or more regions and a task of composing two or more of the classified regions.

As another way, the classification module 21b may select and reclassify only regions related to a predetermined brain disease excluding regions which are not related to the predetermined brain disease from the region which is classified into 95 classes and the hippocampus region which is classified into 13 sub regions again.

Finally, the analysis module 21c acquires the neurodegeneration feature related to the brain of the patient based on the plurality of classified brain regions.

Here, the analysis module 21c may generate a neurodegeneration feature map based on the classified brain region.

Further, referring to FIGS. 8A and 8B, the analysis module 21c may acquire the neurodegeneration feature from the neurodegeneration feature map and the neurodegeneration feature may include a cortical thickness, a volume, a surface area, and a gyrification index.

In the meantime, the analysis module 21c may use at least one of the region, which is classified into 95 classes and the hippocampus region, which is classified into 13 sub regions again by the classification module 21b.

That is, the analysis module 21c may calculate a regional volume from the region, which is classified into 95 classes, a subfield volume from the hippocampus region, which is classified into 13 sub regions again, and a composite region volume from the composite region.

### Amyloid PET image processing unit and tau PET image processing unit

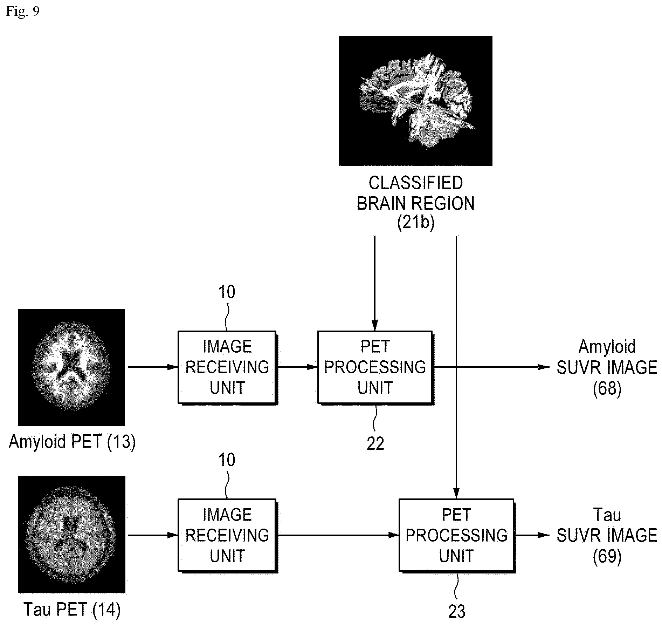

FIG. 9 is a view explaining for a process of acquiring an SUVR image from an amyloid PET image and a tau PET image based on a plurality of classified brain regions, according to the present disclosure.

As described above, the plurality of input images 11 may include the MRI image 12 related to the brain of the patient, the amyloid PET image 13 and the tau PET image 14 related to the brain of the patient.

Further, the SUVR image may include a first SUVR image 68 acquired by the amyloid PET image processing unit 22 and a second SUVR image 69 acquired by the tau PET image processing unit 23.

As described above, the MRI image processing unit 21 classifies the entire region of the brain of the patient into a plurality of regions and the amyloid PET image processing unit 22 and the tau PET image processing unit 23 may acquire the first SUVR image 68 and the second SUVR image 69 from the plurality of classified brain regions.

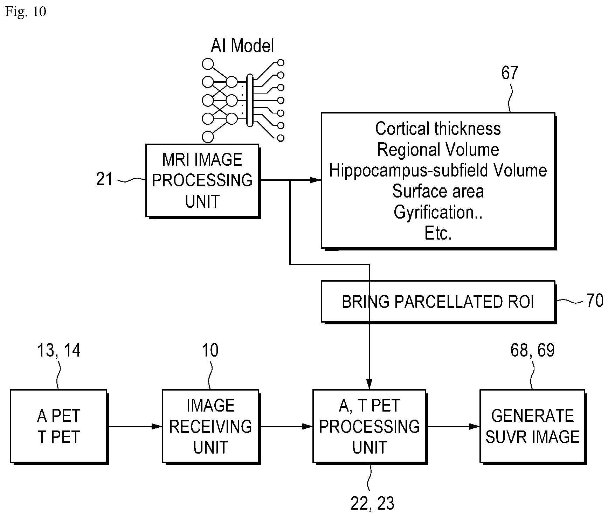

Further, FIG. 10 is a view explaining for a process of acquiring an SUVR image based on region of interest (ROI) information in accordance with an operation of an MRI image processing unit in a plurality of classified brain regions, according to the present disclosure.

Referring to FIG. 10, the amyloid PET image processing unit 22 and the tau PET image processing unit 23 may acquire a first SUVR image 68 and a second SUVR image 69 based on region of interest (ROI) information 70 according to the operation of the MRI image processing unit 21 from the plurality of classified brain regions.

The ROI (Region of Interest) information 70 is utilized during a process of acquiring specific information 67 such as a cortical thickness, a volume, a surface area, a gyrification index, a volume for every region from the classified region, hippocampus subfield volume, or a composite region volume by the MRI image processing unit 21.

Typically, the ROI 70 applied in FIG. 10 may include a cerebellum gray matter region, a cerebellum white matter region, a whole cerebellum region, a pons region, and a brainstem region.

Further, in FIG. 10, the ROI which is used by the amyloid PET image processing unit 22 and the tau PET image processing unit 23 may vary depending on a tracer of the amyloid PET image and the tau PET image.

In the meantime, the amyloid PET image processing unit 22 and the tau PET image processing unit 23 may perform a predetermined pre-processing process.

Here, the pre-processing process may include partial volume correction (PVC) processing and co-registration processing.

Here, the partial volume correction (PVC) processing is performed to correct a spill-out phenomenon that an image is blurred due to a resolution lower than a predetermined reference by the influence of a partial volume effect so that a concentration is measured to be low and a spill-in phenomenon that when the concentration around the region of interest is high, the concentration is measured to be higher than an actual concentration in the region of interest.

Further, the partial volume correction (PVC) processing method may include a geometric transfer matrix method and a Muller-Gartner method.

### Image analysis unit

The image analysis unit 30 may determine whether it is normal or abnormal with respect to a plurality of predetermined biomarkers.

Specifically, the image analysis unit 30 according to the present disclosure may include an MRI image analysis unit 31, an amyloid PET image analysis unit 32, and a tau PET image analysis unit 33.

The MRI image analysis unit 31 makes first determination of whether it is normal or abnormal with respect to a predetermined neurodegeneration feature biomarker.

Further, the amyloid PET image analysis unit 32 makes second determination of whether it is normal or abnormal with respect to a predetermined amyloid PET image biomarker.

Further, the tau PET image analysis unit 33 makes third determination of whether it is normal or abnormal with respect to a predetermined tau PET image biomarker.

### Classifying unit

Next, the classifying unit 40 performs biological classification of the patient related to Alzheimer's disease using the first determination, the second determination, and the third determination.

The biological classification performed by the classifying unit includes first classification indicating that a patient is a normal stage, second classification indicating that the patient is in an early stage of Alzheimer's disease, third classification indicating that the patient corresponds to Alzheimer's disease, fourth classification indicating that the patient has another pathology as well as Alzheimer's disease, and fifth classification indicating that the patient has a pathology other than Alzheimer's disease.

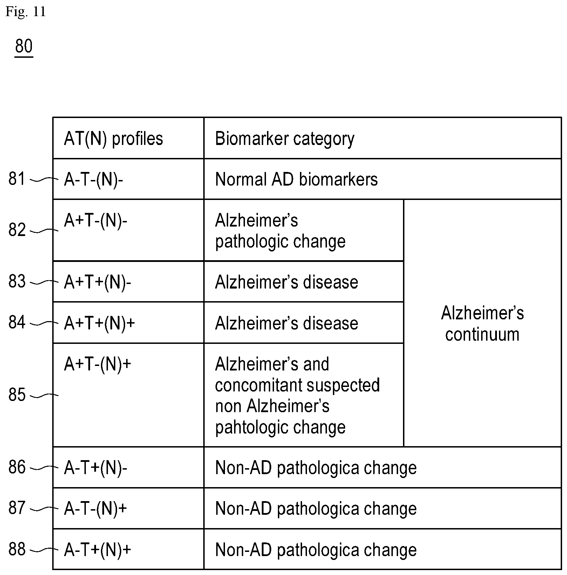

Specifically, FIG. 11 is a table obtained by summarizing biological classification contents performed by a classifying unit as a table, according to the present disclosure.

Referring to FIG. 11, the classifying unit 40 performs first classification indicating that the patient is a normal stage when the first determination is normal with respect to the neurodegeneration feature biomarker, the second determination is normal with respect to the amyloid PET image biomarker, and the third determination is normal with respect to the tau PET image biomarker (81).

Further, the classifying unit 40 performs second classification indicating that the patient corresponds to an early stage of Alzheimer's disease when the first determination is normal with respect to the neurodegeneration feature biomarker, the second determination is abnormal with respect to the amyloid PET image biomarker, and the third determination is normal with respect to the tau PET image biomarker (82).

Further, the classifying unit 40 performs third classification indicating that the patient corresponds to Alzheimer's disease when the first determination is normal with respect to the neurodegeneration feature biomarker, the second determination is abnormal with respect to the amyloid PET image biomarker, and the third determination is abnormal with respect to the tau PET image biomarker (83).

Further, the classifying unit 40 performs third classification indicating that the patient corresponds to Alzheimer's disease when the first determination is abnormal with respect to the neurodegeneration feature biomarker, the second determination is abnormal with respect to the amyloid PET image biomarker, and the third determination is abnormal with respect to the tau PET image biomarker (84).

Further, the classifying unit 40 performs fourth classification indicating that the patient has another pathology as well as a pathology of Alzheimer's disease when the first determination is abnormal with respect to the neurodegeneration feature biomarker, the second determination is abnormal with respect to the amyloid PET image biomarker, and the third determination is normal with respect to the tau PET image biomarker (85).

Further, the classifying unit 40 performs fifth classification indicating that the patient has a pathology other than Alzheimer's disease when the first determination is normal with respect to the neurodegeneration feature biomarker, the second determination is normal with respect to the amyloid PET image biomarker, and the third determination is abnormal with respect to the tau PET image biomarker (86).

Further, the classifying unit 40 performs fifth classification indicating that the patient has a pathology other than Alzheimer's disease when the first determination is abnormal with respect to the neurodegeneration feature biomarker, the second determination is normal with respect to the amyloid PET image biomarker, and the third determination is normal with respect to the tau PET image biomarker (87).

Further, the classifying unit 40 performs fifth classification indicating that the patient has a pathology other than Alzheimer's disease when the first determination is abnormal with respect to the neurodegeneration feature biomarker, the second determination is normal with respect to the amyloid PET image biomarker, and the third determination is abnormal with respect to the tau PET image biomarker (88).

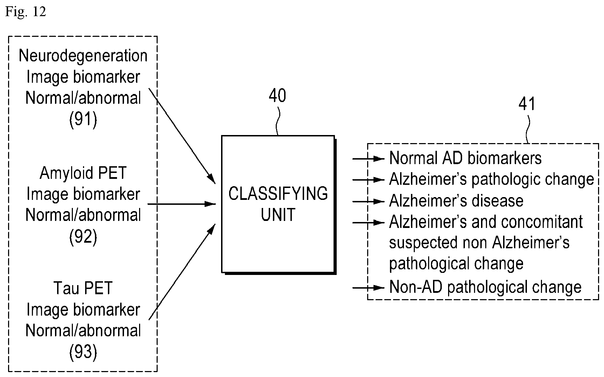

FIG. 12 is a view illustrating a process of biologically classifying Alzheimer's disease by analyzing an MRI image, an amyloid PET image, and a tau PET image related to the brain, according to the present disclosure.

Referring to FIG. 12, the classifying unit 40 receives a first determination result from the MRI image analysis unit 31, a second determination result from the amyloid PET image analysis unit 32, and a third determination result from the tau PET image analysis unit 33.

Further, based on these results, the classifying unit 40 performs biological classification including the first classification indicating that the patient is a normal stage, the second classification indicating that the patient corresponds to an early stage of Alzheimer's disease, the third classification indicating that the patient corresponds to Alzheimer's disease, the fourth classification indicating that the patient has another pathology as well as a pathology of Alzheimer's disease, and the fifth classification indicating that the patient is a pathology other than Alzheimer's disease, as illustrated in FIG. 11, by combining the first determination, the second determination, and the third determination of whether it is normal.

Based on this, it is possible to identify an exact current state of the patient and provide a step in accordance with the current state and a management step in accordance with the possibility of Alzheimer's disease in the future to the patient.

### Method of increasing probability of successful clinical trials by utilizing biological classification of Alzheimer's disease using brain image to screen patient group and normal group First method

The above-described biological classification device and method for Alzheimer's disease using a brain image according to the present disclosure are utilized to screen the patient group and the normal group to increase a probability of successful clinical trials.

That is, the present disclosure may provide a device, a system, and a method which increase a probability of successful clinical trials by utilizing the biological classification device and method for Alzheimer's disease using a brain image to screen a patient group and a normal group.

A result of clinical trials for demonstration of drug efficacy is determined by showing a statistical significance indicating whether to achieve a predicted expected effect for clinical trial participants. However, when the biological classification device and method for Alzheimer's disease using a brain image according to the present disclosure are applied, only Alzheimer's disease patients exactly targeted by new drugs are included as clinical trial subjects so that the probability of successful clinical trials may be increased as much as possible.

First, problems of existing new drug clinical trials will be described in advance.

A result of clinical trials for demonstration of drug efficacy is determined by showing a statistical significance indicating whether to achieve a predicted expected effect for clinical trial participants.

Therefore, in order to prove the statistical significance, a numerical value of an evaluation scale needs to be statistically significantly increased before and after medication or as compared to a placebo group. The higher the predicted increase value, the smaller the number of target subjects and the higher the probability of achieving statistical significance.

In this case, if the predicted increase value is small, the number of target subjects increases as well and the difficulty of statistical proof is increased.

As a result, it is very difficult to increase one step of evaluation scale of the Alzheimer's disease, so that there is a problem in that a possibility of passing the clinical trial is very low.

In the present disclosure, in order to solve the above-described problem, only Alzheimer's disease patients who are exactly targeted by the new drug are included as subjects of the clinical trials to increase a probability of successful clinical trials as much as possible.

One of important failure factors in a new drug development process for central nervous system drugs is the difficulty of screening the correct subjects and screening a drug response group.

Since a response rate to the placebo for the central nervous system drugs is particularly high, an important strategy of increasing the success rate is to reduce the heterogeneity of the subject group and setting a biomarker capable of predicting a drug reactivity.

Further, since it takes a long time to confirm the Alzheimer's disease, a screening test is difficult so that there is a problem in that it is very difficult to include only the Alzheimer's disease patients targeted by new drugs as subjects of clinical trials.

The biological classification device and method for Alzheimer's disease using a brain image proposed by the present disclosure are utilized to screen the patient group and the normal group to increase a probability of successful clinical trials.

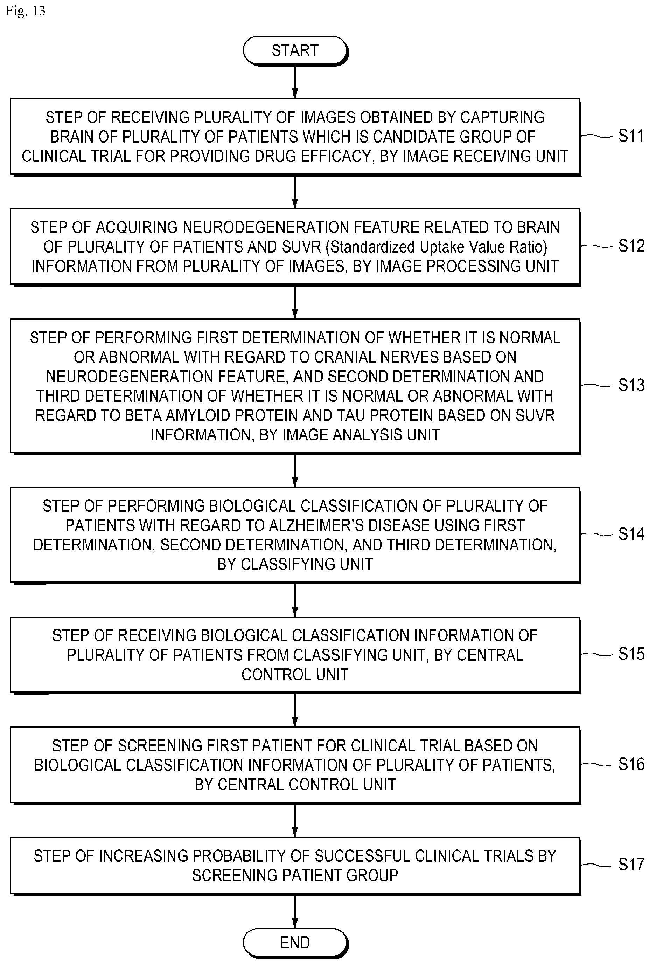

FIG. 13 is a view explaining for a method of increasing a probability of successful clinical trials by utilizing biological classification of Alzheimer's disease using a brain image to screen a patient group and a normal group, according to the present disclosure.

In FIG. 13, a method of increasing a probability of successful clinical trials by screening a patient group using a biological classification device for Alzheimer's disease using a brain image including a central control unit (not illustrated) as well as the image receiving unit 10, the image processing unit 20, the image analysis unit 30, and the classifying unit 40 described above.

Referring to FIG. 13, first, a step S11 of receiving a plurality of images obtained by capturing brains of a plurality of patients which is a candidate group of a clinical trial for proving the drug efficacy by the image receiving unit 10 is performed.

Next, a step S12 of acquiring a neurodegeneration feature with regard to the brains of the plurality of patients and the standardized uptake value ratio (SUVR) image from the plurality of images by the image processing unit 20 is performed.

After the step S12, a step S13 of performing first determination of whether it is normal or abnormal with respect to a predetermined neurodegeneration feature biomarker, second determination of whether it is normal or abnormal with respect to a predetermined amyloid PET image biomarker, and third determination of whether it is normal or abnormal with respect to a predetermined tau PET image biomarker is performed by the image analysis unit 30.

Further, the classifying unit 40 performs biological classification of the plurality of patient related to Alzheimer's disease using the first determination, the second determination, and the third determination (S14).

Next, a step S15 of providing the biological classification information of the plurality of patients from the classifying unit 40 to the central control unit (not illustrates) is performed.

In this case, the central control unit may screen a first patient for the clinical trial based on the biological classification information of the plurality of patients (S16).

After the step S16, the clinical trial is performed on the screened patient group to increase the probability of successful clinical trials (S17).

Accordingly, only the Alzheimer's disease patients who are exactly targeted by the new drugs are included as a clinical trial subject so that the probability of successful clinical trials may be increased as much as possible.

As a result, the biological classification device and method for Alzheimer's disease using a brain image according to the present disclosure are utilized to screen the patient group and the normal group to increase a probability of successful clinical trials.

### Second method

The above-described steps S1 to S4 may be independently performed by the biological classification device for Alzheimer's disease 1 using a brain image or may be applied by providing a separate server (not illustrated) or a separate central control device (not illustrated) to perform the entire operations together with the biological classification device for Alzheimer's disease 1.

The second method explains a method of using a separate server (not illustrated).

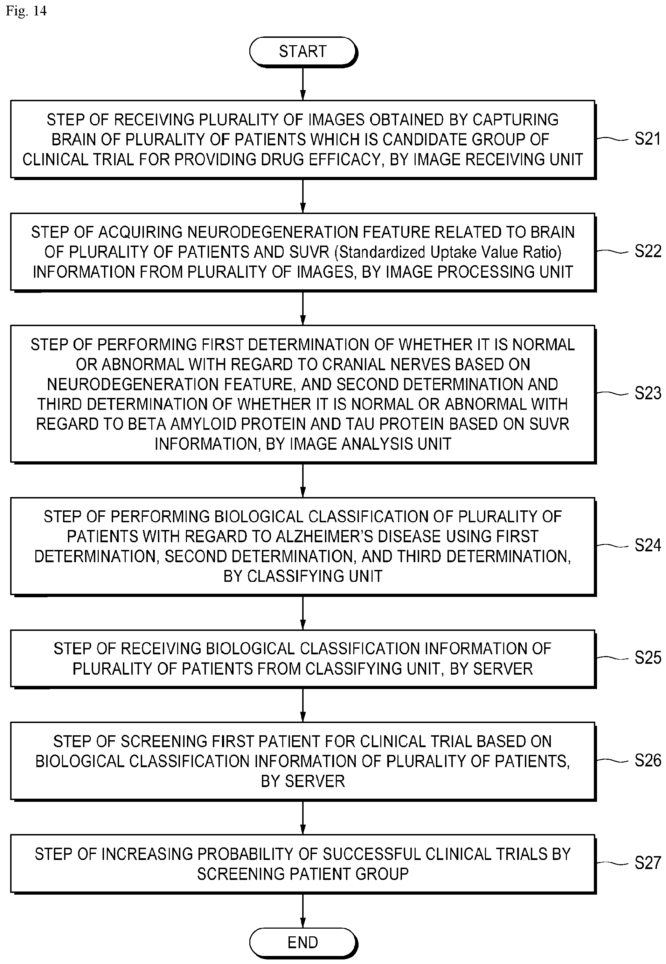

FIG. 14 is a view explaining for another method of increasing a probability of successful clinical trials by utilizing biological classification of Alzheimer's disease using a brain image to screen a patient group and a normal group, according to the present disclosure.

Steps S21 to S24 of FIG. 14 correspond to steps S11 to S14 of FIG. 13 which have been described above so that a detailed description will be omitted for the purpose of simplicity of the description.

After the step S24, a step S25 of providing biological classification information of the plurality of patients from the classifying unit 40 to a server (not illustrated) by wireless or wired communication is performed.

Thereafter, the server may screen a first patient for the clinical trial based on the biological classification information of the plurality of patients (S26).

After the step S26, the clinical trials are performed on the screened patient group to increase the probability of successful clinical trials (S27) and only the Alzheimer's disease patients who are exactly targeted by the new drugs are included as clinical trial subjects to increase the probability of successful clinical trials as much as possible.

As described above, according to the present disclosure, it is possible to provide a device and a method of performing biological classification related to Alzheimer's disease by determining and combining whether it is normal or abnormal with respect to a predetermined biomarker after acquiring a neurodegeneration feature related to the brain of a patient from an MRI image and acquiring a standardized uptake value ratio (SUVR) from an amyloid PET image and a tau PET image.

Specifically, the present disclosure provides a device and a method for performing determination of whether it is normal or abnormal with respect to a neurodegeneration feature biomarker, second determination of whether it is normal or abnormal with respect to an amyloid PET image biomarker, and determination of whether it is normal or abnormal with respect to a tau PET image biomarker, and performing biological classification with respect to Alzheimer's disease based on a combination of three determination results.

Further, the present disclosure provides a biological classification device and method including first classification indicating that a patient is a normal stage, second classification indicating that the patient corresponds to an early stage of Alzheimer's disease, third classification indicating that the patient corresponds to Alzheimer's disease, fourth classification indicating that the patient has another pathology as well as Alzheimer's disease, and fifth classification indicating that the patient has a pathology other than Alzheimer's disease to a user.

Further, the present disclosure provides a device and a method of classifying an entire region of the brain into a plurality of regions based on an MRI image, acquiring a neurodegeneration feature from the plurality of classified brain regions and, at the same time, acquiring an SUVR image from an amyloid PET image and an SUVR image from a tau PET image based on the plurality of classified brain regions to a user.

Further, the present disclosure provides a device and a method of classifying and analyzing a brain of a patient into a plurality of regions based on an MRI image by applying a deep neural network module trained using at least one of a first model trained with a brain image in an axial direction and labelling data, a second model trained with a brain image in a coronal direction and the labelling data, and a third model trained with a brain image in a sagittal direction and the labelling data to a user.

Further, the present disclosure provides a device and a method of acquiring an SUVR image from an amyloid PET image and a tau PET image based on region of interest (ROI) information acquired from an operation of a device of processing an MRI image in a plurality of classified brain regions to a user.

Further, the present disclosure provides a device and a method of performing pre-processing such as partial volume correction (PVC) processing and co-registration processing, with regard to an amyloid PET image and a tau PET image to a user.

Further, the present disclosure provides a device, a system, and a method which increase a probability of successful clinical trials by utilizing biological classification of Alzheimer's disease using a brain image to screen a patient group and a normal group.

A technical object to be achieved in the present disclosure is not limited to the aforementioned effects, and another not-mentioned effects will be obviously understood by those skilled in the art from the description below.

The above-described exemplary embodiments of the present invention may be implemented through various methods. For example, the exemplary embodiments of the present disclosure may be implemented by a hardware, a firm ware, a software, and a combination thereof.

When the exemplary embodiment is implemented by the hardware, the method according to the exemplary embodiment of the present disclosure may be implemented by one or more application specific integrated circuits(ASICs), digital signal processors (DSPs), digital signal processing devices(DSPDs), programmable logic devices(PLDs), field programmable gate arrays(FPGAs), a processor, a controller, a microcontroller, or a microprocessor.

When the exemplary embodiment is implemented by the firmware or the software, the method according to the exemplary embodiment of the present disclosure may be implemented by a module, a procedure, or a function which performs a function or operations described above. The software code is stored in the memory unit to be driven by the processor. The memory unit is located inside or outside the processor and exchanges data with the processor, by various known units.

As described above, the detailed description of the exemplary embodiments of the disclosed present invention is provided such that those skilled in the art implement and carry out the present invention. While the invention has been described with reference to the preferred embodiments, it will be understood by those skilled in the art that various changes and modifications of the present invention may be made without departing from the spirit and scope of the invention. For example, those skilled in the art may use configurations disclosed in the above-described exemplary embodiments by combining them with each other. Therefore, the present invention is not intended to be limited to the above-described exemplary embodiments but to assign the widest scope consistent with disclosed principles and novel features.

The present invention may be implemented in another specific form within the scope without departing from the spirit and essential feature of the present invention. Therefore, the detailed description should not restrictively be analyzed in all aspects and should be exemplarily considered. The scope of the present invention should be determined by rational interpretation of the appended claims and all changes are included in the scope of the present invention within the equivalent scope of the present invention. The present invention is not intended to be limited to the above-described exemplary embodiments but to assign the widest scope consistent with disclosed principles and novel features. Further, claims having no clear quoting relation in the claims are combined to configure the embodiment or may be included as new claims by correction after application.

## Claims

1. A biological classification device for Alzheimer's disease using a brain image, the device comprising:
an image receiving unit which receives a plurality of images obtained by capturing a brain of a subject;
an image processing unit which acquires neurodegeneration feature related to the brain of the subject and SUVR information from the plurality of images;
an image analysis unit which performs first determination of whether it is normal or abnormal with respect to cranial nerves based on the neurodegeneration feature and second determination and third determination of whether it is normal or abnormal with respect to beta amyloid protein and tau protein based on the SUVR information; and
a classifying unit which performs biological classification of the subject related to the Alzheimer's disease using the first determination, the second determination, and the third determination together.

2. The biological classification device according to claim 1, wherein the plurality of images includes an MRI image related to a brain of the subject and an amyloid PET image and a tau PET image related to the brain of the subject.

3. The biological classification device according to claim 2, wherein the SUVR information includes a first SUVR image related to the amyloid PET image and a second SUVR image related to the tau PET image, and the image processing unit classifies the entire region of the brain of the subject into a plurality of regions and acquires the neurodegeneration feature, the first SUVR image, and the second SUVR image from the plurality of classified brain regions.

4. The biological classification device according to claim 3, wherein the image analysis unit includes:
a first image analysis unit which performs first determination of whether it is normal or abnormal with regard to the cranial nerves based on the acquired neurodegeneration feature;
a second image analysis unit which performs second determination of whether it is normal or abnormal with regard to beta amyloid protein based on the first SUVR image; and
a third image analysis unit which performs third determination of whether it is normal or abnormal with regard to tau protein based on the second SUVR image.

5. The biological classification device according to claim 1, wherein the biological classification performed by the classifying unit includes first classification indicating that a subject is a normal stage, second classification indicating that the subject corresponds to an early stage of Alzheimer's disease, third classification indicating that the subject corresponds to Alzheimer's disease, fourth classification indicating that the subject has another pathology as well as Alzheimer's disease, and fifth classification indicating that the subject has a pathology other than Alzheimer's disease.

6. The biological classification device according to claim 5, wherein the classifying unit performs first classification of the subject when the first determination is normal, the second determination is normal, and the third determination is normal, second classification of the subject when the first determination is normal, the second determination is abnormal, and the third determination is normal, third classification of the subject when the first determination is normal, the second determination is abnormal, and the third determination is abnormal and when the first determination is abnormal, the second determination is abnormal, and the third determination is abnormal, fourth classification of the subject when the first determination is abnormal, the second determination is abnormal, and the third determination is normal, and fifth classification of the subject when the first determination is normal, the second determination is normal, and the third determination is abnormal, when the first determination is abnormal, the second determination is normal, and the third determination is normal, and when the first determination is abnormal, the second determination is normal, and the third determination is abnormal.

7. The biological classification device according to claim 3, wherein the image processing unit includes:
a first image processing unit which classifies the entire region of the brain of the subject into a plurality of regions based on the MRI image related to the brain of the subject and acquires the neurodegeneration feature from the plurality of classified brain regions;
a second image processing unit which acquires the first SUVR image from the amyloid PET image related to the brain of the subject, based on the plurality of classified brain regions; and
a third image processing unit which acquires the second SUVR image from the tau PET image related to the brain of the subject, based on the plurality of classified brain regions.

8. The biological classification device according to claim 7, wherein the first image processing unit includes:
a deep neural network module which is trained using at least one of a first model trained with a brain image in an axial direction and labelling data, a second model trained with a brain image in a coronal direction and the labelling data, and a third model trained with a brain image in a sagittal direction and the labelling data;
a classification module which classifies the entire region of the brain of the subject into a plurality of regions based on the MRI image; and
an analysis module which acquires neurodegeneration feature related to the brain of the subject based on the plurality of classified brain region.

9. The biological classification device according to claim 8, wherein the analysis module generates a neurodegeneration feature map based on the classified brain regions and acquires the neurodegeneration information from the neurodegeneration feature map and the neurodegeneration feature includes a cortical thickness, a volume, a surface area, and a gyrification index.

10. The biological classification device according to claim 8, wherein the classification module classifies the entire region of the brain of the subject into a plurality of regions using any one of the first MRI image classified with respect to the axial direction by the first model, the second MRI image classified with respect to the coronal direction by the second model, and the third MRI image classified with respect to the sagittal direction by the third model.

11. The biological classification device according to claim 8, wherein the deep neural network module three-dimensionally reconstructs the MRI image using all the first MRI image classified with respect to the axial direction by the first model, the second MRI image classified with respect to the coronal direction by the second model, and the third MRI image classified with respect to the sagittal direction by the third model.

12. The biological classification device according to claim 8, wherein the classification module classifies the entire region of the brain of the subject into 95 classes based on the MRI image which is three-dimensionally reconstructed and classifies a hippocampus region among the 95 classes into 13 sub regions again.

13. The biological classification device according to claim 12, wherein the classification module reclassifies the region which is classified into 95 classes and the hippocampus region which is classified into 13 sub regions again into a composite region by at least one of a task of additionally classifying into two or more regions and a task of composing two or more of the classified regions.

14. The biological classification device according to claim 12, wherein the classification module selects and reclassifies only regions related to a predetermined brain disease excluding regions which are not related to the predetermined brain disease from the region which is classified into 95 classes and the hippocampus region which is classified into 13 sub regions again.

15. The biological classification device according to claim 13, wherein the analysis module calculates a regional volume from the region which is classified into 95 classes, a subfield volume from the hippocampus region which is classified into 13 sub regions again, and a composite region volume from the composite region.

16. The biological classification device according to claim 7, wherein the second image processing unit and the third image processing unit additionally apply region of interest (ROI) information used for the region classifying operation and neurodegeneration feature operation of the first image processing unit to acquire the first SUVR image and the second SUVR image.

17. The biological classification device according to claim 16, wherein the ROI includes a cerebellum grey matter region, a cerebellum white matter region, a whole cerebellum region, a pons region, and a brainstem region and
the ROI used in the second image processing unit and the third image processing unit varies depending on a tracer of the amyloid PET image and the tau PET image.

18. The biological classification device according to claim 7, wherein the second image processing unit and the third image processing unit perform a predetermined pre-processing process and the pre-processing process includes partial volume correction (PVC) processing and co-registration processing.

19. The biological classification device according to claim 18, wherein the partial volume correction (PVC) processing is performed to correct a spill-out phenomenon that an image is blurred due to a resolution lower than a predetermined reference by the influence of a partial volume effect so that a concentration is measured to be low and a spill-in phenomenon that when the concentration around the region of interest is high, the concentration is measured to be higher than an actual concentration in the region of interest and the partial volume correction (PVC) processing method includes a geometric transfer matrix method and a Muller-Gartner method.

20. A biological classification method for Alzheimer's disease using a brain image, the method comprising:
a first step of receiving a plurality of images obtained by capturing a brain of a subject, by an image receiving unit;
a second step of acquiring a neurodegeneration feature related to the brain of the subject and SUVR information from the plurality of images, by an image processing unit;
a third step of performing first determination of whether it is normal or abnormal with respect to cranial nerves based on the neurodegeneration feature and second determination and third determination of whether it is normal or abnormal with respect to beta amyloid protein and tau protein based on the SUVR information, by an image analysis unit; and
a fourth step of performing biological classification of the subject related to the Alzheimer's disease using the first determination, the second determination, and the third determination together, by the classifying unit.

21. The biological classification method according to claim 20, wherein the plurality of images includes an MRI image related to a brain of the subject and an amyloid PET image and a tau PET image related to the brain of the subject.

22. The biological classification method according to claim 21, wherein the SUVR information includes a first SUVR image related to the amyloid PET image and a second SUVR image related to the tau PET image and
the image processing unit classifies the entire region of the brain of the subject into a plurality of regions and acquires the neurodegeneration feature, the first SUVR image, and the second SUVR image from the plurality of classified brain regions.

23. The biological classification method according to claim 22, wherein the third step includes:
a 3-1 step of performing first determination of whether it is normal or abnormal with regard to the cranial nerves based on the acquired neurodegeneration feature, by a first image analysis unit of the image analysis unit;
a 3-2 step of performing second determination of whether it is normal or abnormal with regard to the beta amyloid protein based on the first SUVR image, by a second image analysis unit of the image analysis unit; and
a 3-3 step of performing third determination of whether it is normal or abnormal with regard to the tau protein based on the second SUVR image, by a third image analysis unit of the image analysis unit.

24. The biological classification method according to claim 20, wherein in the fourth step, the biological classification performed by the classifying unit includes first classification indicating that a subject is a normal stage, second classification indicating that the subject corresponds to an early stage of Alzheimer's disease, third classification indicating that the subject corresponds to Alzheimer's disease, fourth classification indicating that the subject has another pathology as well as Alzheimer's disease, and fifth classification indicating that the subject has a pathology other than Alzheimer's disease.

25. The biological classification method according to claim 24, wherein in the fourth step, the classifying unit performs first classification of the subject when the first determination is normal, the second determination is normal, and the third determination is normal, second classification of the subject when the first determination is normal, the second determination is abnormal, and the third determination is normal, third classification of the subject when the first determination is normal, the second determination is abnormal, and the third determination is abnormal and when the first determination is abnormal, the second determination is abnormal, and the third determination is abnormal, fourth classification of the subject when the first determination is abnormal, the second determination is abnormal, and the third determination is normal, and fifth classification of the subject when the first determination is normal, the second determination is normal, and the third determination is abnormal, when the first determination is abnormal, the second determination is normal, and the third determination is normal, and when the first determination is abnormal, the second determination is normal, and the third determination is abnormal.

26. The biological classification method according to claim 22, wherein the second step includes:
a 2-1 step of classifying the entire region of the brain of the subject into a plurality of regions based on the MRI image related to the brain of the subject and acquiring the neurodegeneration feature from the plurality of classified brain regions, by a first image processing unit of the image processing unit;
a 2-2 step of acquiring the first SUVR image from the amyloid PET image related to the brain of the subject, based on the plurality of classified brain regions, by a second image processing unit of the image processing unit; and
a 2-3 step of acquiring the second SUVR image from the tau PET image related to the brain of the subject, based on the plurality of classified brain regions, by a third image processing unit of the image processing unit.

27. The biological classification method according to claim 26, wherein the 2-1 step includes:
training a deep neural network module of the first image processing unit using at least one of a first model trained with a brain image in an axial direction and labelling data, a second model trained with a brain image in a coronal direction and the labelling data, and a third model trained with a brain image in a sagittal direction and the labelling data;
classifying an entire region of a brain of the subject based on the MRI image into a plurality of regions, by a classification module of the first image processing unit; and
acquiring a neurodegeneration feature related to the brain of the subject, based on the plurality of classified brain regions, by an analysis module of the first image processing unit.

28. The biological classification method according to claim 27, wherein the analysis module generates a neurodegeneration feature map based on the classified brain regions and acquires the neurodegeneration information from the neurodegeneration feature map and the neurodegeneration feature includes a cortical thickness, a volume, a surface area, and a gyrification index.

29. The biological classification method according to claim 27, wherein the classification module classifies the entire region of the brain of the subject into a plurality of regions using any one of the first MRI image classified with respect to the axial direction by the first model, the second MRI image classified with respect to the coronal direction by the second model, and the third MRI image classified with respect to the sagittal direction by the third model.

30. The biological classification method according to claim 27, wherein the deep neural network module three-dimensionally reconstructs the MRI image using all the first MRI image classified with respect to the axial direction by the first model, the second MRI image classified with respect to the coronal direction by the second model, and the third MRI image classified with respect to the sagittal direction by the third model.

31. The biological classification method according to claim 26, wherein the second image processing unit and the third image processing unit additionally apply region of interest (ROI) information used for the region classifying operation and a neurodegeneration feature operation of the first image processing unit to acquire the first SUVR image and the second SUVR image.

32. The biological classification method according to claim 31, wherein the ROI includes a cerebellum grey matter region, a cerebellum white matter region, a whole cerebellum region, a pons region, and a brainstem region and the ROI used in the second image processing unit and the third image processing unit varies depending on a tracer of the amyloid PET image and the tau PET image.

33. The biological classification method according to claim 26, wherein the second image processing unit and the third image processing unit perform a predetermined pre-processing process and the pre-processing process includes partial volume correction (PVC) processing and co-registration processing.

34. A method of increasing a probability of successful clinical trials by screening a subject group using a biological classification device for Alzheimer's disease using a brain image which includes an image receiving unit, an image processing unit, an image analysis unit, a classifying unit, and a central control unit, the method comprising:
a first step of receiving a plurality of images obtained by capturing brains of a plurality of subjects which is a candidate group of a clinical trial for proving a drug efficacy, by the image receiving unit;
a second step of acquiring a neurodegeneration feature related to the brain of the plurality of subjects and SUVR information from the plurality of images, by the image processing unit;
a third step of performing first determination of whether it is normal or abnormal with respect to cranial nerves based on the neurodegeneration feature and second determination and third determination of whether it is normal or abnormal with respect to beta amyloid protein and tau protein based on the SUVR information, by the image analysis unit;
a fourth step of performing biological classification of the plurality of subjects related to the Alzheimer's disease using the first determination, the second determination, and the third determination together, by the classifying unit;
a fifth step of providing the biological classification information of the plurality of subjects from the classifying unit to the central control unit; and
a sixth step of screening a first subject for the clinical trial based on the biological classification information of the plurality of subjects, by the central control unit.

35. A method of increasing a probability of successful clinical trials by screening a subject group using a biological classification device for Alzheimer's disease using a brain image which includes an image receiving unit, an image processing unit, an image analysis unit, and a classifying unit, and a server which communicates with the biological classification device for Alzheimer's disease, the method comprising:
a first step of receiving a plurality of images obtained by capturing brains of a plurality of subjects which is a candidate group of a clinical trial for proving a drug efficacy, by the image receiving unit;
a second step of acquiring a neurodegeneration feature related to the brain of the plurality of subjects and SUVR information from the plurality of images, by the image processing unit;
a third step of performing first determination of whether it is normal or abnormal with respect to cranial nerves based on the neurodegeneration feature and second determination and third determination of whether it is normal or abnormal with respect to beta amyloid protein and tau protein based on the SUVR information, by the image analysis unit;
a fourth step of performing biological classification of the plurality of subjects related to the Alzheimer's disease using the first determination, the second determination, and the third determination together, by the classifying unit;
a fifth step of providing the biological classification information of the plurality of subjects from the classifying unit to the server; and
a sixth step of screening a first subject for the clinical trial based on the biological classification information of the plurality of subjects, by the server.
